# EUROPEAN PATENT APPLICATION

(11) **EP 3 123 933 A1**
(43) Date of publication of application: **01.02.2017**
(21) Application number: 16175447.8
(22) Date of filing: 21.06.2016
(51) Int. Cl.: A61B 5/00, A61B 5/0408, A61B 5/0492

(54) **PHYSIOLOGY SENSING DEVICE AND INTELLIGENT TEXTILE**

(30) Priority: 29.07.2015 TW 104124566
(71) Applicant: Far Eastern New Century Corporation, Taipei (TW)
(72) Inventor: LAI, Hsin-Kai, Taoyuan City 320 (TW); HUNG, Wei-Che, Taoyuan City 320 (TW); WU, Yu-June, Taoyuan City 320 (TW); HSU, Jeffrey-Fu, Taoyuan City 320 (TW)
(74) Representative: D Young & Co LLP

(57) **Abstract**

A physiology sensing device and an intelligent textile are provided. The physiology sensing device includes a fabric substrate and a conductive coating layer. The conductive coating layer is embedded from a side of the fabric substrate and levelled with the fabric body, and a thickness of the conductive coating layer is not larger than a thickness of the fabric substrate. The conductive coating layer includes a hydrophobic adhesive and a plurality of conductive particles distributing therein. The physiology sensing device receives variation of a signal of body potential on skin surface. Then a long range signal receiver receives the signal, the signal is shown on a monitory system so as to perform long distance monitoring.

## Description

This application claims priority to Taiwanese Application Serial Number 104124566, filed July 29, 2015.

The present invention relates to a physiology sensing device. More particularly, the present invention relates to a physiology sensing device capable of integrating into fabric to form a functional textile to detect physical status of a user.

With the improvement of economy, science and living standard, the understanding of health evolves as well. The current measuring standard of health is not only expressive by the absence of physical disease or pain. It is desired to know in advance if there is any abnormal change occurring in the data representing physical status by a physiology monitoring system such that abnormal health status can be detected in an early stage.

To solve this problem, Taiwanese Patent Publication No. 528593 discloses a device for monitoring physical status and the method of device monitoring. The signals associated to physical status from inside and outside the body are collected as information. The signals are detected by skin-fit device, and the detected data are retrieved for safety precaution. The variety of detecting devices disclosed in the patent application is metal detecting components, such that the sensing interface, which contacts the user's skin, is cool. This coolness makes the user feel uncomfortable especially in a cold weather. Furthermore, these detected devices are metallic electronic components with certain volume and dimension. When wearing the device, the foreign particles make it even more uncomfortable. In addition, when the detecting electronic devices are disposed in the fabric, the fabric cannot be washed by water if stains happen. To wash this type of fabric, the electronic components have to be removed and reassembled after cleaning. This process renders the fabric cleaning inconvenient.

To further overcome the above issues, Taiwanese Patent Publication No. I274576 discloses a physiology sensing fabric and monitor system. In this disclosure, fabric electrodes replace known metal made sensing elements. The fabric electrodes and the fabric layer in this patent publication are made by conductive threads and non-conductive threads through weaving, knitting or non-woven as a one-piece product. Although the fabric electrodes made of conductive threads can improve the hardness of metal-made electronic components and reduce the uncomfortable feeling of the coolness, the conductive threads are fragile. After a period of time, because of the friction between the clothes and the body or the corrosion caused by perspiration, the conductive threads are likely to break down or have much higher resistance leading to the loss of conductivity. Furthermore, in this patent application, the conductive electrodes are said to be launderingable. However, there was not specific embodiment to support this property. According to this patent application, one of ordinary skill in the art can understand that the conductive threads have a diameter ranging from a few micrometers to tens of micrometers, and the surface conductive layer covering the conductive threads is even thinner, which is prone to damage by external force, leading to the loss of conductive function. Therefore, although the conductive threads in this patent application may pass one or twice tests under water, this fabric may not pass the standard laundering test in the associated field.

Hence, there is an urgent call for a conductive electrode that is comfortable to wear, strong in structure and able to pass standard laundering test in this field.

Accordingly, the instant disclosure provides a physiology sensing device for detecting variation of a signal of body potential on skin surface.

Another aspect of the instant disclosure provides an intelligent textile with the aforementioned physiology sensing device embedded on an inner side of the intelligent textile. The intelligent textile can sense variation of a signal of body potential on skin surface, and in addition, a more comfortable wearing experience.

In order to achieve the previously described purpose, according to an embodiment of the instant disclosure, a physiology sensing device for sensing variation of a signal of body potential on skin surface is provided. The physiology sensing device includes a fabric substrate and a conductive coating layer. The conductive coating layer is embedded from a side of the fabric substrate and levelled with the fabric substrate, and a thickness of the conductive coating layer is not larger than a thickness of the fabric substrate. The conductive coating layer includes a hydrophobic adhesive and a plurality of conductive particles distributing therein.

According to an embodiment of the instant disclosure, the conductive coating layer completely embedded into the fabric substrate, and the side of the conductive coating layer and the side of the fabric substrate are co-planar.

According to an embodiment of the instant disclosure, a portion of the conductive coating layer projects out of the surface of the fabric substrate.

According to an embodiment of the instant disclosure, the hydrophobic adhesive is selected from a group consisting of polyurethane, polysiloxane, polyethylene terephthalate, polyacrylate and the combination thereof.

According to an embodiment of the instant disclosure, the hydrophobic adhesive is polyurethane.

According to an embodiment of the instant disclosure, the plurality of conductive particles are metal conductive particles.

According to an embodiment of the instant disclosure, a main ingredient of the metal conductive particles is selected from a group consisting of gold, silver, copper, metal oxide and the combination thereof.

According to an embodiment of the instant disclosure, the plurality of conductive materials are non-metal conductive particles.

According to an embodiment of the instant disclosure, a main ingredient of the non-metal conductive materials is selected from a group consisting of carbon nanotube, carbon black, carbon fiber, graphene, conductive polymer and the combination thereof.

According to an embodiment of the instant disclosure, the plurality of conductive particles in the conductive coating layer is 20-70 wt%.

According to an embodiment of the instant disclosure, the plurality of conductive particles in the conductive coating layer is 30-50 wt%.

According to an embodiment of the instant disclosure, the thickness of the conductive coating layer embedded in the fabric substrate is 10-50 µm.

According to an embodiment of the instant disclosure, a thickness of the conductive coating layer projecting out of the fabric substrate is less than 40 µm.

According to an embodiment of the instant disclosure, the conductive coating layer is formed with a plurality of holes.

According to an embodiment of the instant disclosure, the conductive coating layer is a continuous pattern layer.

According to an embodiment of the instant disclosure, the aforementioned physiology sensing device is embedded on an inner side of an intelligent textile.

According to an embodiment of the instant disclosure, the intelligent textile further includes a control unit electrically connected to the physiology sensing device to convert the body potential change signal to a physiology signal.

The physiology sensing device of the instant disclosure can be combined with clothes from the inner side so as to sense the body potential change from the user's skin layer. Subsequently, the body potential change signals are transferred to a signal emitter from a known signal transmitting path, and the signals are transmitted from the signal emitter. Finally, after the signals are received by a long range signal receiver, the signals are shown on the monitor system so as to monitor from a long distance.

It is to be understood that both the foregoing general description and the following detailed description are by examples, and are intended to provide further explanation of the invention as claimed.

The invention can be more fully understood by reading the following detailed description of example embodiments, with reference made to the accompanying drawings as follows:
Fig. 1 is a cross-sectional view of a physiology sensing device in accordance with an example embodiment of the instant disclosure;
Fig. 2 is a cross-sectional view of a physiology sensing device in accordance with another example embodiment of the instant disclosure; and
Fig. 3 is a schematic diagram showing an intelligent textile embedded with a physiology sensing device on an inner side of the intelligent textile in accordance with an example embodiment of the instant disclosure.

Reference will now be made in detail to the present embodiments of the invention, examples of which are illustrated in the accompanying drawings. Wherever possible, the same reference numbers are used in the drawings and the description to refer to the same or like parts.

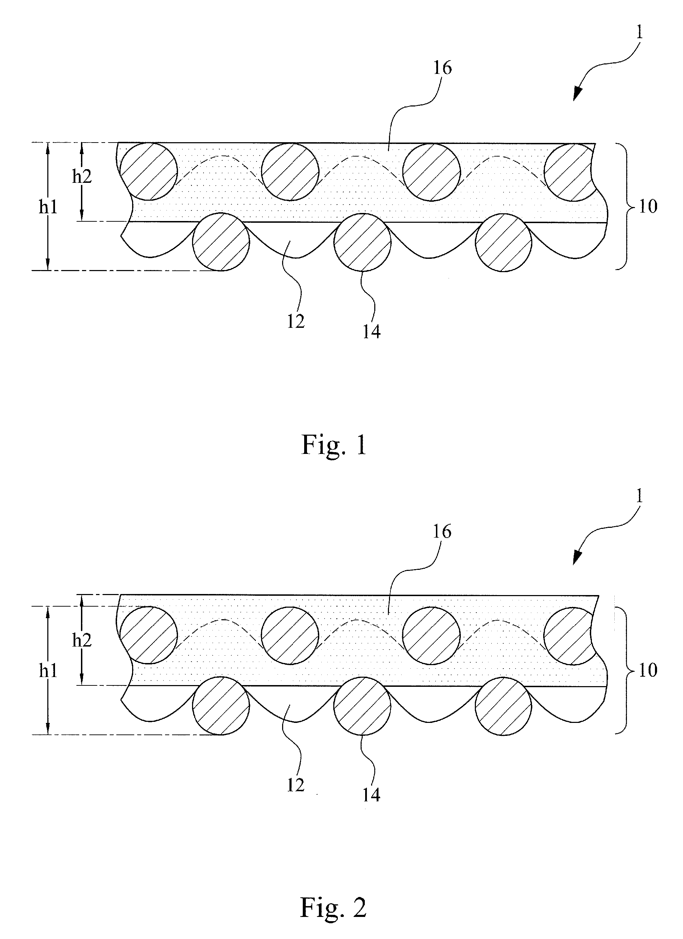

Please refer to Fig. 1, illustration a cross-sectional view of a physiology sensing device in accordance with an embodiment of the instant disclosure. The physiology sensing device 1 of the instant disclosure constitutes of a fabric substrate 10 and a conductive coating layer 16 embedded therein. In this embodiment, the fabric substrate 10 is a weaving fabric (i.e., plain weaving fabric). It is made of a plurality of weft threads 12 and a plurality of warp threads 14 interlacing one another. As a result, the fabric substrate 10 has a thickness h1 formed by the interlacing threads. The conductive coating layer 16 is embedded from one side of the fabric substrate 10 into the fabric substrate 10. The conductive coating layer 16 fills in the gaps in between the interlacing threads of the fabric substrate 10 and is integrated as one piece.

In this embodiment, the conductive coating layer 16 is completely merged into the fabric substrate 10 and interlocked with the fabric substrate 10. The upper side of the conductive coating layer 16 is coplanar with the upper side of the fabric substrate 10, and the lower side of the conductive coating layer 16 is inside the fabric substrate 10. As a result, the contour of the conductive coating layer 16 is substantially levelled with the upper side of the fabric substrate 10. In this embodiment, the thickness h2 of the conductive coating layer 16 is not larger than the thickness h1 of the fabric substrate 10.

Please refer to Fig. 2, illustrating a cross-sectional view of a physiology sensing device in accordance with another embodiment of the instant disclosure. In this embodiment, the physiology sensing device 1 of the instant disclosure constitutes of a fabric substrate 10 and a conductive coating layer 16 embedded in the fabric substrate 10. The difference lies on the conductive coating layer 16 partially embedded in the fabric substrate 10. That is, a thickness portion of the conductive coating layer 16 projects out of the fabric substrate 10.

The thickness projecting out of the fabric substrate 10 of previously described conductive coating layer 16 is not limited in the instant disclosure. However, for the sake of comfort when the physiology sensing device 1 contacting wearer skin, the projected thickness portion is preferably not larger than 40 micrometers, in another embodiment not larger than 30 micrometers, and in still another embodiment not larger than 20 micrometers.

In the previously described fabric substrate 10 is made by weaving. However, one of ordinary skill in the art should understand from the instant disclosure that the fabric substrate 10 may be made by knitting. However, for less deformation and overall thickness, it is preferably to use weaving fabric as the fabric substrate 10. The types and forms of the weaving fabric that is compatible in the instant disclosure is not limited, as long as the conductive coating layer 16 can be embedded in the fabric, and the structural strength is not compromised.

According to the physiology sensing device 1 of the instant disclosure, the conductive coating layer 16 is completely or partially embedded in the fabric substrate 10. As a result, the fabric substrate 10 further provides the conductive coating layer 16 a physical support and protection, such that the structural strength of the conductive coating layer 16 is enhanced. As a result, the physiology sensing device 1 of the instant disclosure provides a structural enhanced effect similar to a composite material. The thickness of the conductive coating layer 16 embedded in the fabric substrate 10 is preferably 10 to 50 micrometers, or more preferably 20 to 40 micrometers. If the thickness of the conductive coating layer 16 embedded in the fabric substrate 10 is less than 10 micrometers, it easily results in peeling of the conductive coating layer 16 from the surface of the fabric substrate 10. In contrast, if the thickness of the conductive coating layer 16 embedded in the fabric substrate 10 is more than 50 micrometers, resistance unevenness is likely to occur.

The conductive coating layer 16 is made of a hydrophobic adhesive and a plurality of conductive particles distributed among the conductive coating layer 16. The material of hydrophobic adhesive applicable in the instant disclosure includes but not limited to polyurethane (PU), silicone resin, polyethylene terephthalate (PET), polyacrylates and the like. The material of the conductive particles applicable in the instant disclosure includes non-metal materials, metal materials or the combination thereof. The non-metal materials include but not limited to carbon nanotubes (CNT), carbon black, carbon fiber, graphene and conductive polymers (e.g., poly(3,4-ethylenedioxythiophene) (PEDOT), polyacrylonitrile, (PAN) or the like). Carbon nanotubes provides the most preferably result. The metal materials includes but not limited to gold, silver, copper and metal oxide (e.g., indium tin oxdie (ITO)) and the like.

In order to have better body potential sensing effect, the ratio of the conductive particles in the conductive coating layer 16 is preferably 20-70 wt%, more preferably 30-50 wt%. The physiology sensing device 1 of the instant disclosure has a preferable surface resistance ranging between 100-300 Ω/square meter, more preferably 150-250 Ω/ square meter.

The conductive coating layer 16 can be embedded in the fabric substrate 10 by any known approaches. For example, the hydrophobic adhesive is dissolved in a solvent, and the conductive particles are distributed in the solution to form a conductive coating solution. Subsequently, the conductive coating solution coats on the fabric substrate and immerses into the fabric substrate to form a conductive coating layer. Finally, the conductive coating layer is dried to be completely dry. As a result, the physiology sensing device 1 of the instant disclosure is obtained. The coating method in the instant disclosure is not limited. However, to achieve an even spreading and flat surface, known printing skill may be executed, for example, gravure printing, screen printing, relief printing and slot coating or the like, and the instant disclosure is not limited thereto. Alternatively, the conductive coating solution is applied to a piece of release paper to form a conductive coating layer. Following that, it is preliminarily dried. Subsequently, before the conductive coating layer is not completely dried, it is roller press-fit to the fabric substrate. Finally, the release paper is peeled off, and the conductive coating layer is completely dried. As a result, the physiology sensing device 1 of the instant disclosure is obtained.

When applying the physiology sensing device of the instant disclosure, it can be combined at the inner side of fabric (or clothes) such that a side with unexposed conductive coating layer is combined with the fabric, while a side with exposed conductive coating layer contacts wearer's skin directly. The combination means is not limited in the instant disclosure, as long as the physiology sensing device of the instant disclosure can be combined from the inner side of the fabric, it is applicable. The combination means include but not limited to adherence attachment or sewing.

To further increase the comfort of the physiology sensing device of the instant disclosure when contacting wearer's skin, the physiology sensing device of the instant disclosure may be formed with a plurality of holes. The holes go through the physiology sensing device of the instant disclosure. The deposition number and dimension of the holes are not limited in the instant disclosure. As long as perspiration steam and body heat can be exhausted, the holes are applicable to the instant disclosure. Alternatively, the conductive coating layer may be a substantially continuous pattern embedded in the fabric substrate. That is, the conductive coating layer does not cover the entire surface of the fabric substrate. In addition, a plurality of holes may be formed in the conductive coating layer. As a result, the perspiration steam from the wearer can be exhausted from the holes instead of accumulating at the position where the physiology sensing device of the instant disclosure contacts the skin.

In order to transmit the signal of body potential change of the wearer detected by the physiology sensing device of the instant disclosure, a conductive terminal is formed on the physiology sensing device of the instant disclosure. The deposition means and number of the conductive terminal may vary according to the requirement, and the instant disclosure is not limited thereto.

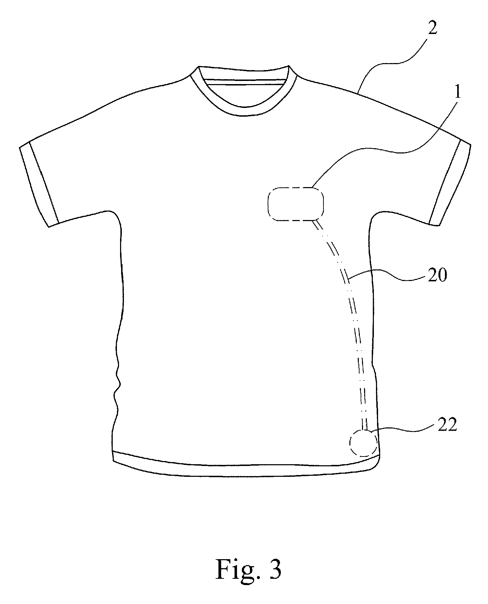

Please refer to Fig. 3, illustrating a schematic diagram of an intelligent textile having the physiology sensing device of the instant disclosure in accordance with an embodiment of the instant disclosure. The physiology sensing device 1 of the instant disclosure is mounted at the inner side of the clothes 2 and electrically connected to a control unit 22 through a conductive wire 20.

The material of the conductive wire 20 is not limited in the instant disclosure. As long as the wire can transmit the signal of the body potential change detected by the physiology sensing device of the instant disclosure to the control unit, it is applicable to the instant disclosure. For example, the same structural design as the physiology sensing device of the instant disclosure can be used, but lower resistance is required. For example, the surface resistance is not larger than 10 Ω, more preferably 0.01-1Ω.

The type and format of the control unit 22 is not limited in the instant disclosure. As long as the electronic device can convert the body potential change signal to physiology information, it is applicable to the instant disclosure. The control unit 22 may further integrate with a wireless signal emitting unit, such that the electronic signals can be transmitted. The wireless signal emitting unit may also be independent from the control unit, stands alone and electrically connected to the control unit.

Because the conductive coating layer 16 is completely or partially embedded in the fabric substrate 10, the physiology sensing device 1 of the instant disclosure is relatively thinner. The thickness of the physiology sensing device of the instant disclosure is substantially similar or the same as the fabric substrate 10, and the softness of the physiology sensing device of the instant disclosure is not compromised. As a result, when the physiology sensing device 1 of the instant disclosure is mounted at the inner side of the clothes 2, the wearer does not feel a distinct presence of foreign particle so as to provide a better comfort in wearing the clothes.

When a wearer wears the intelligent textile, the body potential change signal generated by the wearer, for example, the body potential change because of hear beat or muscle contraction or the like, will be detected by the physiology sensing device 1 of the instant disclosure. Subsequently, the signals are transmitted to the control unit 22 through the conductive wire 20. The control unit 22 will further convert the body potential change signal to physiology information. Finally, the physiology information is transmitted to a receiving device, for example, any known intelligent portable device like mobile phone, watch, bracelet, tablet or the like, by wireless signal transmission.

### Sample Preparation

### Embodiment 1

Commercially available polyurethane and n-butyl acetate (n-BAC) are formulated to form a polyurethane coating solution (solid content 30 wt%). The carbon nanotube is added to the polyurethane coating solution. The addition of carbon nanotube is measured by weight ratio 1 against 5 (carbon nanotube against polyurethane coating solution) and allows thorough homogenisation so as to obtain the conductive coating solution. The conductive coating solution is printed on a commercially available plain weave fabric by a 200 mesh screen and dried under hot air at 150ºC so as to remove the solvent and form a conductive coating layer embedded in the plain weave fabric. As a result, the physiology sensing device (thickness of the conductive coating layer: 40 micrometers; thickness of the conductive coating layer embedded in the plain weave fabric: 10 micrometers) of the instant disclosure is obtained. In this condition, the percentage of the carbon nanotube in the conductive coating layer is 40 wt%. The resulting physiology sensing device has a surface resistance approximately 250 Ω/square meter (ambient temperature 26ºC, ambient relative humidity 60-65%).

### Embodiment 2

The same preparation condition is applied as Embodiment 1, but a 150 mesh screen was used instead. As a result, a physiology sensing device having surface resistance of 150 Ω/square meter is obtained. (A screen having not larger mesh number has larger eyes, and the thickness of the coating layer will be thicker.)

### Comparison sample 1

Known metal conductive threads were used to make a plain weave fabric by a conventional weaving machine. The measured surface resistance was 250 Ω/square meter.

### Comparison sample 2

Known metal conductive threads were used to make a plain weave fabric by a conventional weaving machine, but the ratio of metal threads was higher than Sample 1. The measured surface resistance was 150 Ω/square meter.

### Laundering Test

Laundering test was conducted according to AATCC method 135 (laundering 50 times). The surface resistance of the samples was measured after laundering test by ohmmeter (purchased from Mitsubishi Electric, catalogue number MCP-T610).The results after laundering test are shown in Table 1.

**TABLE 1**

| Test Sample | Surface Resistance (Ω/square meter) | |
|---|---|---|
| | Before laundering | After Laundering |
| Embodiment 1 | 250 | 262 |
| Embodiment 2 | 150 | 153 |
| Comparison sample 1 | 250 | 699 |
| Comparison sample 2 | 150 | 651 |

According to the results, after the laundering test, the surface resistance of the physiology sensing device of the instant disclosure did not increase too much, and it was still in an operable range. However, after laundering test, the surface resistance of the comparison samples representing conventional conductive threads increased dramatically, and it was beyond an operable range.

### Sample Bending Test

Sample bending test was conducted by folding the samples in half and 2 kg weight was added atop for 1 minute. This process was repeated 500 times. The surface resistance of the samples was measured after bending test by ohmmeter (purchased from Mitsubishi Electric, catalogue number MCP-T610). The results after bending test are shown in Table 2.

**TABLE 2**

| Test Sample | Surface Resistance at Bent Region (Ω/square meter) | |
|---|---|---|
| | Before Bending | After Bending |
| Embodiment 1 | 180 | 202 |
| Embodiment 2 | 130 | 161 |
| Comparison sample 1 | 180 | >1M |
| Comparison sample 2 | 130 | >1M |

According to the results, after the bending test, the surface resistance at the bent region of the physiology sensing device of the instant disclosure did not increase too much, and it was still in an operable range. However, after bending test, the surface resistance at the bent region of the comparison samples representing conventional conductive threads increased dramatically, and it was beyond an operable range, which was almost at a state of circuit interruption (resistance > 1 M).

### Wearing Resistance Test

Sample wearing resistance test was using JIS-L-1096 method as a reference. The wearing resistance of the samples were tested under Martindale machine for fraction of 1,000 times. The surface resistance of the samples was measured after bending test by ohmmeter (purchased from Mitsubishi Electric, catalogue number MCP-T610).The results after bending test are shown in Table 3.

**TABLE 3**

| Test Sample | Surface Resistance at Worn Region (Ω/square meter) | |
|---|---|---|
| | Before Wearing | After Wearing |
| Embodiment 1 | 250 | 271 |
| Embodiment 2 | 150 | 165 |
| Comparison Sample 1 | 250 | 1,780 |
| Comparison Sample 2 | 150 | 1,560 |

According to the results, after the wearing test, the surface resistance at the worn region of the physiology sensing device of the instant disclosure did not increase too much, and it was still in an operable range. However, after wearing test, the surface resistance at the worn region of the comparison samples representing conventional conductive threads increased dramatically, and it was beyond an operable range.

Based on the results of the experiment, the physiology sensing device of the instant disclosure has better quality than conventional device, it passes the laundering test, bending test and wearing resistance test, showing wider application in industry.

## Claims

1. A physiology sensing device (1) for detecting variation of a signal of body potential on skin surface, comprising:
a fabric substrate (10); and
a conductive coating layer (16), the conductive coating layer (16) including a hydrophobic adhesive and a plurality of conductive particles distributing therein;
wherein the conductive coating layer (16) is embedded from a side of the fabric substrate (10) and levelled with the fabric substrate (10), and a thickness (h2) of the conductive coating layer (16) is not larger than a thickness (h1) of the fabric substrate (10).

2. The physiology sensing device of claim 1, wherein the conductive coating layer (16) completely merged and embedded into the fabric substrate (10), and the side of the conductive coating layer (16) is flush with the side of the fabric substrate (10).

3. The physiology sensing device of claim 1, wherein a portion of the conductive coating layer (16) projects out of the surface of the fabric substrate (10).

4. The physiology sensing device of claim 3, wherein a thickness (h2) of the conductive coating layer (16) projecting out of the fabric substrate (10) is less than 40 µm.

5. The physiology sensing device of any one of the preceding claims, wherein the fabric substrate (10) is a weaving fabric.

6. The physiology sensing device of any one of the preceding claims, wherein the hydrophobic adhesive is selected from the group consisting of polyurethane, polysiloxane, polyethylene terephthalate, polyacrylate and the combination thereof.

7. The physiology sensing device of claim 6, wherein the hydrophobic adhesive is polyurethane.

8. The physiology sensing device of any one of the preceding claims, wherein the plurality of conductive particles are metal conductive particles.

9. The physiology sensing device of any one of claims 1 to 7, wherein the plurality of conductive particles are non-metal conductive particles.

10. The physiology sensing device of any one of the preceding claims, wherein the plurality of conductive particles in the conductive coating layer (16) is 20-70 wt%.

11. The physiology sensing device of any one of the preceding claims, wherein the thickness (h2) of the conductive coating layer (16) embedded in the fabric substrate (10) is 10-50 µm

12. The physiology sensing device of any one of the preceding claims, wherein the conductive coating layer (16) is formed with a plurality of holes.

13. The physiology sensing device of any one of claims 1 to 11, wherein the conductive coating layer (16) is a continuous pattern layer.

14. An intelligent textile (2), wherein the physiology sensing device (1) of claim 1 is mounting on an inner side of the intelligent textile.

15. The intelligent textile of claim 14, further comprising: a control unit (22) electrically connected to the physiology sensing device (1) to convert the variation of body potential signal to a physiology signal.
